(19) Europäisches Patentamt
European Patent Office
Office européen des brevets

(11) Veröffentlichungsnummer: **0 236 898 B1**

# (12) EUROPÄISCHE PATENTSCHRIFT

(45) Veröffentlichungstag der Patentschrift: **27.11.91**

(51) Int. Cl.5: **C07C 21/18**, C07C 17/20

(21) Anmeldenummer: **87102913.8**

(22) Anmeldetag: **02.03.87**

(54) **Verfahren zur Herstellung von 3-Fluor-2-(fluormethyl)-propen und die neue Verbindung 3-Fluor-2-(chlormethyl)-propen.**

(30) Priorität: **13.03.86 DE 3608380**

(43) Veröffentlichungstag der Anmeldung:
**16.09.87 Patentblatt 87/38**

(45) Bekanntmachung des Hinweises auf die Patenterteilung:
**27.11.91 Patentblatt 91/48**

(84) Benannte Vertragsstaaten:
**DE FR GB IT**

(56) Entgegenhaltungen:
**EP-A- 0 120 575**
**GB-A- 1 088 925**
**US-A- 3 655 786**
**US-A- 3 739 036**

**CHEMICAL ABSTRACTS, Band 48, Nr. 3, 10. Februar 1954, Zusammenfassungsnr. 1238h, Columbus, Ohio, US; E. GRYSZKIEWICZ-TROCHIMOWSKI et al.: "Some aliphatic fluorine compounds. VI.", & BULL. SOC. CHIM. FRANCE 1953, 123-124 (Kat. D)**

**HOUBEN-WEYL "Methoden der organischen Chemie", 4. Auflage, Band V/3, Seiten 153-154, Georg Thieme Verlag, Stuttgart;**

(73) Patentinhaber: **BAYER AG**

**W-5090 Leverkusen 1 Bayerwerk(DE)**

(72) Erfinder: **Hagemann, Hermann, Dr.**
**Kandinskystrasse 52**
**W-5090 Leverkusen(DE)**
Erfinder: **Baasner, Bernd, Dr.**
**Hamberger Strasse 27d**
**W-5090 Leverkusen 3(DE)**

**EP 0 236 898 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 3-Fluor-2-(fluormethyl)-propen (siehe Formel (I))

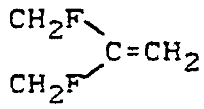

(I),

das auch als Difluorisobutylen bezeichnet werden kann, sowie die neue Verbindung 3-Fluor-2-(chlormethyl)-propen (siehe Formel (II))

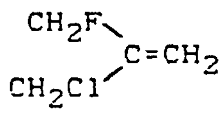

(II),

die auch als Fluor-chlor-isobutylen bezeichnet werden kann.

3-Fluor-2-(fluormethyl)-propen ist ein wertvolles Zwischenprodukt für Synthesen in der organischen Chemie, z.B. auf den Gebieten der Farbstoffe, Pharmazeutika, Pflanzenschutzmittel, Kunststoffe, sowie Textil- und Kunststoffhilfsmittel. Besonders geeignet ist dieses spezielle Olefin zur Synthese Fluor enthaltender Nitroaliphaten, die wiederum als Vorprodukte zur Herstellung von Herbiziden eingesetzt werden können (siehe US-PS 4 533 776).

Es ist bekannt, daß man 3-Fluor-2-(fluormethyl)-propen (I) als unerwünschtes Nebenprodukt infolge pyrolytischer Abspaltung von Fluorwasserstoff und Formaldehyd in einer Ausbeute von lediglich 12,7 % erhält, wenn man Pentaerythrit-tribromhydrin (III) mit Kaliumfluorid zu der entsprechenden Trifluorverbindung umzusetzen versucht (siehe Bull. Soc. Chim. France 1953, 123/124). Diese Reaktion wird durch folgende Gleichung (1) illustriert:

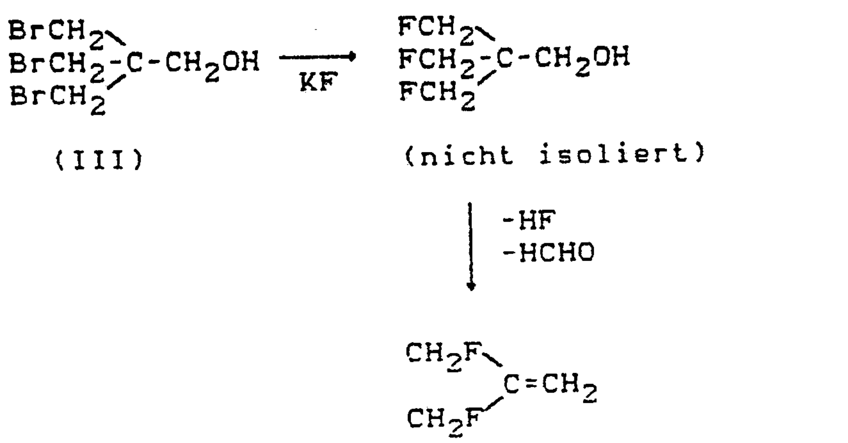


(III) (nicht isoliert)

(1)

(I) 12,7 % Ausbeute

Mit dieser Synthesefolge besteht natürlich keine Möglichkeit zur Herstellung von 3-Fluor-2-(fluormethyl)-propen (I) in technisch-industriellem Maßstab.

Es wurde nun ein Verfahren zur Herstellung von 3-Fluor-2-(fluormethyl)-propengefunden, das dadurch gekennzeichnet ist, daß man 3-Chlor-2-(chlormethyl)-propen mit einem Fluorierungsmittel umsetzt. Als Zwischenstufe wird dabei das bisher unbekannte 3-Fluor-2-(chlormethyl)-propen (II) durchlaufen, das bei geeigneter Reaktionsführung, z.B. Dosierung des Fluorierungsmittels in stöchiometrischer Menge, auch zum Hauptprodukt der Reaktion werden kann, sonst aber immer als abtrennbares Nebenprodukt mit anfällt.

Das erfindungsgemäße Verfahren wird durch die folgende Gleichung (2) beispielhaft erläutert:

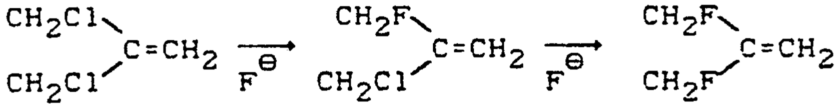


(IV) (II) (I) (2)

Die Ausgangsverbindung 3-Chlor-2-(chlormethyl)-propen (IV) ist eine bekannte Verbindung. Sie entsteht beispielsweise als Zwangsanfallsprodukt bei der Allylchlorierung von Isobutylen zum Methallylchlorid, die großtechnisch durchgeführt wird. Sie kann aber auch gemäß EP-A 159 508 gezielt aus Methallylchlorid durch Umsetzung mit Sulfurylchlorid in der Flüssigphase in guter Ausbeute hergestellt werden.

Als Fluorierungsmittel für das erfindungsgemäße Verfahren kommen beispielsweise übliche Fluorierungsmittel in Frage. Im Hinblick auf ihre gute technische und preisgünstige Zugänglichkeit sind beispielsweise Kaliumfluorid, Kaliumhydrogenfluorid, Natriumfluorid, Natriumhydrogenfluorid, Kalciumfluorid und Magnesiumfluorid, gegebenenfalls auch in Gemischen untereinander und in Gemischen mit kleinen Mengen Cäsiumfluorid, geeignet. Besonders gute Ausbeuten erhält man im allgemeinen beim Einsatz von Kaliumfluorid. In das erfindungsgemäße Verfahren können auch wasserhaltige oder nicht besonders vorgetrocknete Fluoride eingesetzt werden. In diesen Fällen verwendet man zweckmäßigerweise ein Lösungsmittel und entfernt das eingebrachte Wasser aus dem Reaktionsgemisch durch Destillation eines Teils des Lösungsmittels, bei der dann das Wasser mitentfernt wird.

Theoretisch werden für die Umsetzung von 3-Chlor-2-(chlormethyl)-propen (IV) zu 3-Fluor-2-(fluormethyl)-propen (I) 2 Äquivalente Fluorierungsmittel benötigt. Bevorzugt wird das Fluorierungsmittel im Überschuß eingesetzt, beispielsweise in einer Menge von 2,01 bis 2,5 Äquivalenten, vorzugsweise in einer Menge von 2,03 bis 2,2 Äquivalenten, jeweils bezogen auf die eingesetzte Menge der Verbindung (IV). Größere Überschüsse führen im allgemeinen nur zu wenig höheren Umsätzen und zu nur wenig verringerten Gehalten der Monofluorverbindung (II) im Reaktionsgemisch. Der Einsatz geringerer Mengen an Fluorierungsmittel, beispielsweise 0,8 bis 1,5 Äquivalente Fluorierungsmittel, bezogen auf die eingesetzte Menge der Verbindung (IV), führt im Reaktionsgemisch zu einem starken Ansteigen des Gehaltes an der Monofluorverbindung (II) und wird dann angewendet, wenn man gezielt diese Verbindung herzustellen wünscht.

Das erfindungsgemäße Verfahren kann bei verschiedenen Temperaturen durchgeführt werden, beispielsweise bei solchen im Bereich von 80 bis 300 °C. Bevorzugt sind Temperaturen zwischen 110 und 180 °C. Die erfindungsgemäße Umsetzung kann bei Normaldruck oder erhöhtem Druck durchgeführt werden. Erhöhter Druck ist vor allem dann vorteilhaft, wenn man ein Lösungsmittel verwenden möchte, das bei Normaldruck unterhalb der beabsichtigten Reaktionstemperatur siedet. Die erfindungsgemäße Umsetzung kann sowohl kontinuierlich als auch diskontinuierlich durchgeführt werden.

Im allgemeinen ist es vorteilhaft, die erfindungsgemäße Umsetzung in Gegenwart eines Lösungsmittels durchzuführen. Geeignete Lösungsmittel sind solche, die unter den Reaktionsbedingungen inert oder weitgehend inert sind. In Frage kommen beispielsweise Ether, z.B. Ethylpropylether, n-Butylethylether, Di-n-butylether, Diisobutylether, Diisoamylether, Diisopropylether, Anisol, Phenetol, Cyclohexylmethylether, Diethylether, Tetrahydrofuran, Dioxan, Thioanisol, β,β'-Dichlordiethylether; Polyethylenglykolether wie Ethylglykoldimethylether, -diethylether, -di-n-propylether, -diisopropylether, -di-n-butylether, -diisobutylether, -di-sek.-butylether, -di-tert.-butylether, Diethylenglykoldimethylether, -diethylether, -di-n-propylether, -diisopropylether, -di-sek.-butylether, -di-tert.-butylether; analoge Diether des Triethylenglykols und des Tetraethylenglykols; Diethylenglykolmonomethylether, Diethylenglykolmonoethylether, Triethylenglykolmonobutylether; Ethylenglykol, Diethylenglykol, Triethylenglykol, Tetraethylenglykol, 1,2-Propandiol, 1,3-Propandiol; Sulfoxide wie Dimethylsulfoxid, Diethylsulfoxid, Dimethylsul fon, Diethylsulfon, Methylethylsulfon, Tetramethylensulfon (Sulfolan); N-Methylpyrrolidon, Dimethylformamid, Hexamethylphosphorsäuretriamid, N,N'-Dimethylpropylenharnstoff, N-Methyl-caprolactam; Benzol, Toluol; Acetonitril und hinsichtlich Qualität und Quantität beliebige Gemische dieser Lösungsmittel. Bevorzugte Lösungsmittel sind Diethylenglykol, Triethylenglykol, Tetraethylenglykol und N-Methylpyrrolidon; besonders bevorzugt Tri- und Tetraethylenglykol. Bezogen auf die Ausgangsverbindung (IV) können beliebige Mengen Lösungsmittel eingesetzt werden. Geeignet sind z.B. solche Lösungsmittelmengen, daß eine 10 bis 80 gew.-%ige Mischung der Ausgangsverbindung (IV) im jeweiligen Lösungsmittel oder Lösungsmittelgemisch vorliegt. Bevorzugt beträgt die Konzentration dieser Mischung 35 - 65 Gew.-%.

Zusätze von Katalysatoren, beispielsweise Kronenethern, haben im allgemeinen nur einen geringen Einfluß auf den Umsatz der Ausgangsverbindung (IV) und die Ausbeute an 3-Fluor-2-(fluormethyl)-propen

(I). Deshalb ist der Einsatz von Katalysatoren, beispielsweise Kronenethern, zwar möglich, aber nicht bevorzugt.

Nach der erfindungsgemäßen Umsetzung liegt im allgemeinen ein Reaktionsgemisch vor, das neben dem difluorierten Produkt (I) noch monofluoriertes Produkt (II), unumgesetztes Ausgangsprodukt (IV), das aus dem Fluorierungsmittel entstandene entsprechende Chlorid und gegebenenfalls im Überschuß eingesetztes Fluorierungsmittel und gegebenenfalls zugesetztes Lösungsmittel enthält. Die Aufarbeitung derartiger Reaktionsgemische kann auf einfache Weise durchgeführt werden, beispielsweise indem man das difluorierte Produkt, das monofluorierte Produkt, das unumgesetzte Ausgangsprodukt und gegebenenfalls das Lösungsmittel in der Reihenfolge ihrer Siedepunkte auseinanderdestilliert. Im allgemeinen geht dabei zunächst das Difluorprodukt, dann das Monofluorprodukt und dann das unumgesetzte Ausgangsprodukt über. Falls man bei der Umsetzung ein Lösungsmittel einsetzen möchte, wählt man dessen Siedepunkt vorteilhafterweise so, daß es nicht zu nahe bei einer der anderen Komponenten siedet. Insbesondere beim Einsatz der bevorzugten Lösungsmittel kann man in vielen Fällen das difluorierte Produkt, das monofluorierte Produkt und unumgesetztes Ausgangsprodukt aus dem Lösungsmittel abdestillieren und letzteres als Destillationsrückstand abtrennen. Diese destillative Trennung kann man bei Normaldruck oder vermindertem Druck durchführen. Geeignete Drucke sind im allgemeinen solche von Normaldruck bis etwa 10 mbar. Eine zu starke Druckerniedrigung kann bewirken, daß die Destillate nur mit größerem Kühlaufwand aufgefangen werden können und sind deshalb weniger vorteilhaft. Das aus dem Fluorierungsmittel entstandene Chlorid und gegebenenfalls im Überschuß eingesetztes Fluorierungsmittel kann man vor der Destillation durch Filtration entfernen (bevorzugt) oder nach der Destillation im Destillationssumpf belassen (weniger bevorzugt). Unumgesetztes Ausgangsprodukt (IV), monofluoriertes Produkt (II), Lösungsmittel und im Überschuß eingesetztes Fluorierungsmittel kann man getrennt oder gemeinsam einem weiteren Ansatz zur Durchführung des erfindungsgemäßen Verfahrens zuführen. Man kann auch kontinuierlich arbeiten, beispielsweise indem man das difluorierte Produkt (I) und das aus dem Fluorierungsmittel entstandene Chlorid laufend aus dem Reaktionsgemisch entfernt und entsprechende Mengen an Ausgangsprodukt (IV) und Fluorierungsmittel nachdosiert. Das auf diese oder eine andere Weise abgetrennte Reaktionsprodukt (I) kann gegebenenfalls durch eine weitere Destillation gereinigt werden, falls dies gewünscht wird.

Das erfindungsgemäße Verfahren gestattet die Herstellung von 3-Fluor-2-(fluormethyl)-propen (I) auf einfache Weise und in guten Ausbeute und mit guten Reinheiten. Die Erzielbarkeit dieser Ergebnisse ist ausgesprochen überraschend, da gemäß dem angegebenen Stand der Technik, ausgehend von der gesättigten Verbindung (III), nur sehr geringe Mengen an 3-Fluor-2-(fluormethyl)-propen (I) erhalten werden können. Beim erfindungsgemäßen Einsatz der ungesättigten Ausgangsverbindung (IV) war, bedingt durch die vorhandene reaktive Doppelbindung, mit dem Ablauf von mehr Nebenreaktionen zu rechnen, als beim Einsatz der gesättigten Ausgangsverbindung (III), z.B. auch mit Oligomerisierungen und Verharzungen.

Im Rahmen der vorliegenden Erfindung wurde weiterhin das neue 3-Fluor-2-(chlormethyl)-propen der Formel (II) gefunden.

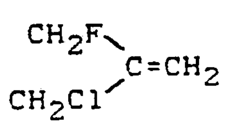 (II)

Diese Verbindung kann auch als Fluor-chlor-isobutylen bezeichnet werden.

Die Herstellung der Verbindung (II) und auch Bedingungen, unter denen die Verbindung (II) in überwiegendem Maße erhalten werden kann, ist bereits weiter oben beschrieben worden.

Die neue Verbindung der Formel (II) kann wie oben beschrieben in die bekannte Verbindung der Formel (I) überführt werden, die gemäß der US-PS 4 533 776 ein Zwischenprodukt zur Herstellung von Herbiziden ist.

Beispiele

Beispiel 1

1,22 kg (21 Mol) Kaliumfluorid wurden in 1,75 l Tetraethylenglykol vorgelegt. Zur Entfernung geringer Mengen eingeschleppten Wassers wurden zunächst 150 ml Tetraethylenglykol zusammen mit diesem Wasser bei einem Druck von 15 mbar abdestilliert. Anschließend wurden 1,25 kg (10 Mol) 3-Chlor-2-(chlormethyl)-propen (IV) hinzugefügt und 3 Stunden zum Rückfluß erhitzt. Anschließend wurden die

flüchtigen Bestandteile bei 15 mbar aus dem Lösungsmittel in eine gut gekühlte Vorlage abdestilliert. Das erhaltene Rohdestillat wurde über eine Kolonne bei einem Druck von 1013 mbar fraktioniert. Es wurden erhalten:

Fraktion 1:

390 g, Siedepunkt 62 - 64 °C.
Gehalt an 3-Fluor-2-(fluormethyl)-propen (I): 98,3 % (nach GC).

Zwischenfraktion:

50 g, Siedepunkt 65 - 98 °C.
Gehalt an (I): 39,6 %. (nach GC), Gehalt an 3-Fluor-2-(chlormethyl)-propen (II): 57,1 %. (nach GC).

Fraktion 2:

240 g, Siedepunkt 98 - 100 °C.
Gehalt an (II): 94,3 % (nach GC).

Rückstand:

305 g Ausgangsmaterial (IV) mit einem Gehalt an (IV) von 87,8 % (nach GC).

Eine redestillierte Probe der Fraktion 2 (Siedepunkt 99-100 °C, Reinheit nach GC 99,4 %) hatte einen Brechungsindex von $n_D^{20}$ = 1,4237.

In nachfolgenden Ansätzen wurde der Rückstand, die Fraktion 2 und die Zwischenfraktion ohne weitere Behandlung wieder eingesetzt.

Beispiel 2

Es wurden 2,44 kg Kaliumfluorid in 3,5 l Tetraethylenglykol vorgelegt und zur Entfernung geringer Mengen eingeschleppten Wassers 300 ml Tetraethylenglykol zusammen mit diesem Wasser bei einem Druck von 15 mbar abdestilliert. Bei einer Badtemperatur von 135 °C und einer Innentemperatur von 120 ± 3 °C wurden dann 2,5 kg 3-Chlor-2-(chlormethyl)-propen (IV) innerhalb von 40 Minuten zudosiert. Gleichzeitig wurden über eine 30 cm-Füllkörperkolonne die bei diesen Bedingungen flüchtigen Anteile bei 1013 mbar abdestilliert. Anschließend wurde noch 4 Stunden nachreagieren gelassen und bei einem Druck von 20 mbar der Rest der flüchtigen Anteile überdestilliert. Das so erhaltene Rohdestillat wurde über eine Kolonne bei einem Druck von 1013 mbar fraktioniert. Es wurden 1,01 kg der Verbindung (I) mit einem Siedepunkt von 61 - 64 °C und einer Reinheit von 97,4 % (nach GC) erhalten.

Beispiele 3 - 11

Es wurde verfahren wie im Beispiel 1, jedoch wurden die Reaktionszeit, die Lösungsmittel, die Konzentrationsverhältnisse im Reaktionsgemisch und die molaren Verhältnisse von Fluorierungsmittel zu Ausgangsmaterial variiert. Alle Versuche wurden bei Rückflußtemperatur durchgeführt. Es wurde jeweils 1 Mol (125 g) 3-Chlor-2-(chlormethyl)-propen (IV) eingesetzt. Das erhaltene Rohdestillat wurde gaschromatographisch auf seine Zusammensetzung hin untersucht. Die Ergebnisse sind aus der folgenden Tabelle ersichtlich.

Tabelle

| Bsp. Nr. | Kaliumfluorid [g] | Lösungsmittel [ml] | Reaktionszeit [h] | Rohdestillat [g] | Zusammensetzung des Rohdestillats [Gew.-%] Verbindung (I) | (II) | (IV) |
|---|---|---|---|---|---|---|---|
| 3 | 122 | Diethylenglykol 160 | 3 | 67 | 59,9 | 31,8 | 7,1 |
| 4 | 122 | Triethylenglykol 160 | 3 | 71 | 61,7 | 30,7 | 6,2 |
| 5 | 122 | N-Methylpyrrolidon 160 | 3 | 104 | 26,1 | 47,0 | 24,9 |
| 6 | 122 | Tetraethylenglykol 160 | 5 | 61 | 82,8 | 14,3 | 1,9 |
| 7 | 122 | Tetraethylenglykol 160 | 1 | 86 | 45,4 | 40,0 | 12,0 |
| 8 | 122 | Tetraethylenglykol 250 | 3 | 68 | 87,4 | 9,8 | 1,9 |
| 9 | 174 | Tetraethylenglykol 160 | 3 | 63 | 78,3 | 14,6 | 4,1 |
| 10 | 122 | Tetraethylenglykol 110 | 3 | 64 | 66,8 | 23,9 | 6,8 |
| 11 | 122 plus 1,5 g [18] Krone-6 | Tetraethylenglykol 160 | 3 | 73 | 90,8 | 6,3 | 1,8 |

## Patentansprüche

1. Verfarhen zur Herstellung von 3-Fluor-2-(fluormethyl)-propen, dadurch gekennzeichnet, daß man 3-Chlor-2-(chlormethyl)-propen mit einem Fluorierungsmittel umsetzt.

2. Verfahren nach Anspruch 1, dadurch gekennzeichnet, daß man als Fluorierungsmittel Kaliumfluorid, Kaliumhydrogenfluorid, Natriumfluorid, Natriumhydrogenfluorid, Kalciumfluorid und/oder Magnesiumfluorid, gegebenenfalls im Gemisch mit kleinen Mengen Cäsiumfluorid einsetzt.

3. Verfahren nach Ansprüchen 1 und 2, dadurch gekennzeichnet, daß man bezogen auf 3-Chlor-2-(chlormethyl)-propen 2,01 bis 2,5 Äquivalente Fluorierungsmittel einsetzt.

4. Verfahren nach Ansprüchen 1 bis 3, dadurch gekennzeichnet, daß man die Umsetzung bei einer Temperatur im Bereich von 80 bis 300 °C durchführt.

5. Verfahren nach Ansprüchen 1 bis 4, dadurch gekennzeichnet, daß man die Umsetzung in Gegenwart eines Lösungsmittels durchführt.

6. Verfahren nach Anspruch 5, dadurch gekennzeichnet, daß man als Lösungsmittel Diethylenglykol, Triethylenglykol, Tetraethylenglykol und/oder N-Methylpyrrolidon einsetzt.

7. Verfahren nach Ansprüchen 1 bis 6, dadurch gekennzeichnet, daß man aus dem nach der Umsetzung vorliegenden Reaktionsgemisch das 3-Fluor-2-(fluormethyl)-propen, das 3-Fluor-2-(chlormethyl)-propen und das 3-Chlor-2-(chlormethyl)-propen in der Reihenfolge ihrer Siedepunkte abdestilliert und das so erhaltene 3-Fluor-2-(fluormethyl)-propen gegebenenfalls durch eine weitere Destillation reinigt.

8. Die chemische Verbindung 3-Fluor-2-(chlormethyl)-propen der Formel

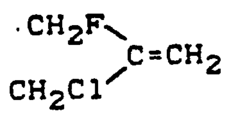

.

## Claims

1. Process for the preparation of 3-fluoro-2-(fluoromethyl)-propene, characterised in that 3-chloro-2-(chloromethyl)-propene is reacted with a fluorinating agent.

2. Process according to Claim 1, characterised in that potassium fluoride, potassium hydrogen fluoride, sodium flouride, sodium hydrogen fluoride, calcium fluoride and/or magnesium fluoride, if appropriate mixed with small amounts of caesium fluoride, is/are employed as the fluorinating agent.

3. Process according to Claims 1 and 2, characterised in that 2.01 to 2.5 equivalents of fluorinating agent, based on 3-chloro-2-(chloromethyl)-propene, are employed.

4. Process according to Claims 1 to 3, characterised in that the reaction is carried out at a temperature in the range from 80 to 300 °C.

5. Process according to Claims 1 to 4, characterised in that the reaction is carried out in the presence of a solvent.

6. Process according to Claim 5, characterised in that diethylene glycol, triethylene glycol, tetraethylene glycol and/or N-methylpyrrolidone is/are employed as the solvent.

7. Process according to Claims 1 to 6, characterised in that the 3-fluoro-2-(fluoromethyl)-propene, the 3-fluoro-2-(chloromethyl)-propene and the 3-chloro-2-(chloromethyl)-propene are distilled off in the sequence of their boiling points from the reaction mixture present after the reaction and, if appropriate, the 3-fluoro-2-(fluoromethyl)-propene thus obtained is purified by further distillation.

8. The chemical compound 3-fluoro-2-(chloromethyl)-propene of the formula

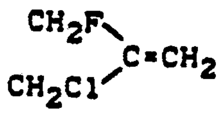 .

## Revendications

1. Procédé de préparation du 3-fluoro-2-(fluorométhyl)-propène, caractérisé en ce que l'on fait réagir le 3-chloro-2-(chlorométhyl)-propène avec un agent fluorant.

2. Procédé selon la revendication 1, caractérisé en ce que l'on utilise en tant qu'agent fluorant le fluorure de potassium, le bifluorure de potassium, le fluorure de sodium, le bifluorure de sodium, le fluorure de calcium et/ou le fluorure de magnésium, éventuellement en mélange avec des petites quantités de fluorure de caesium.

3. Procédé selon les revendications 1 et 2, caractérisé en ce que l'on utilise de 2,01 à 2,5 équivalents d'agent fluorant par rapport au 3-chloro-2-(chlorométhyl)-propène.

4. Procédé selon les revendications 1 à 3, caractérisé en ce que l'on effectue la réaction à une température dans l'intervalle de 80 à 300 °C.

5. Procédé selon les revendications 1 à 4, caractérisé en ce que l'on effectue la réaction en présence d'un solvant.

6. Procédé selon la revendication 5, caractérisé en ce que l'on utilise comme solvant le diéthylène-glycol, le triéthylène-glycol, le tétraéthylène-glycol et/ou la N-méthylpyrrolidone.

7. Procédé selon les revendications 1 à 6, caractérisé en ce que l'on sépare du mélange de réaction obtenu, par distillation, dans l'ordre de leurs points d'ébullition, le 3-fluoro-2-(fluorométhyl)-propène, le 3-fluoro-2-(chlorométhyl)-propène et le 3-chloro-2-(chlorométhyl)-propène et le cas échéant on purifie le 3-fluoro-2-(fluorométhyl)-propène ainsi obtenu par une nouvelle distillation.

8. Le composé chimique 3-fluoro-2-(chlorométhyl)-propène de formule